# EUROPEAN PATENT APPLICATION

(11) **EP 4 365 281 A1**
(43) Date of publication of application: **08.05.2024**
(21) Application number: 22833339.9
(22) Date of filing: 28.06.2022
(51) Int. Cl.: C12N 5/0775, C12N 5/10

(54) **METHOD FOR PRODUCING CARTILAGE CELL STRUCTURE DERIVED FROM IPS CELL**

(30) Priority: 29.06.2021 JP 2021107656
(71) Applicant: SAGA UNIVERSITY, Saga-shi, Saga 840-8502 (JP)
(72) Inventor: NAKAYAMA, Koichi, Saga-shi, Saga 840-8502 (JP); NAKAMURA, Anna Maria, Saga-shi, Saga 840-8502 (JP)
(74) Representative: J A Kemp LLP
(86) International application number: PCT/JP2022/026536
(87) International publication number: WO 2023/277195

(57) **Abstract**

A method for producing a three-dimensional structure of cartilage cells, the method being characterized by comprising layering cell aggregates that have been produced by culturing a mesenchymal stem cell using a culture medium for inducing the differentiation into a cartilage cell on the 9th or 10th day after the starting of the culture.

## Description

### TECHNICAL FIELD

The present invention relates to a method for producing a cartilage cell structure. In particular, the present invention relates to a method for producing a cartilage cell structure using Bio 3D Printer.

### BACKGROUND ART

Fabrication of a cartilage from human induced pluripotent stem cells (iPS cells) has conventionally been studied, and methods, such as a culture method for fabricating cartilage tissue from human iPS cell-derived cartilage cells without using a scaffold (Yamashita A. et al., Stem Cell Reports, 2015 (Non-patent literature 1)), a method for inducing a somite or sclerotome from iPS cells (Matsuda S. et al., Nature, 2020 (Non-patent literature 2)), and a method for inducing hypertrophic cartilage cells from proliferating cartilage cells (Pretemer Y. et al., Stem Cell Reports, 2021 (Non-patent literature 3)), have been established.

Since the cartilage cells fabricated in this way generate extracellular matrix in large amount, they may serve as a clinically applicable tool.

### CITATION LIST

### Non-patent literature

[Non-patent literature 1] Yamashita A. et al., Stem Cell Reports, 2015
[Non-patent literature 2] Matsuda S. et al., Nature, 2020
[Non-patent literature 3] Pretemer Y. et al., Stem Cell Reports, 2021

### SUMMARY OF INVENTION

### Technical Problem

However, in order to further discover the clinical advantage of the cartilage cells differentiated from iPS cells, three-dimensional fabrication of the cartilage cells into cartilage tissue has been a problem.

### Solution to Problem

As a result of diligent study to solve the above problem, the present inventor succeeded in obtaining a highly functional three-dimensional structure of cartilage cells by forming cell aggregates (spheroids) by culturing mesenchymal stem cells, in particular mesenchymal stem cells derived from iPS cells, using a medium that induces differentiation into cartilage cells, and stacking these cell aggregates to form a structure at a predetermined timing after the start of culture using the induction differentiation medium, thereby completing the present invention.

Thus, the present invention is as follows.
(1) A method for producing a three-dimensional structure of cartilage cells, the method comprising stacking cell aggregates obtained by culturing mesenchymal stem cells using a medium that induces differentiation into cartilage cells, on the 9th or 10th day after the start of culture.
(2) The method according to (1), wherein the mesenchymal stem cells are derived from pluripotent stem cells.
(3) The method according to (2), wherein the pluripotent stem cells are induced pluripotent stem cells.
(4) The method according to any one of (1)-(3), wherein the medium that induces differentiation contains a platelet-derived growth factor.
(5) The method according to any one of (1)-(4), wherein the culture is performed in the presence of a transforming growth factor from the 6th day after the start of culture.
(6) A method for producing cartilage tissue, the method comprising further culturing a three-dimensional structure produced by the method according to any one of (1)-(5) in the presence of bone morphogenetic protein.
(7) A three-dimensional structure of cartilage cells, which is obtained by stacking cell aggregates obtained by culturing mesenchymal stem cells using a medium that induces differentiation into cartilage cells, on the 9th or 10th day after the start of culture.
(8) Cartilage tissue obtained by further culturing the three-dimensional structure according to (7) in the presence of bone morphogenetic protein.

### Advantageous Effects of Invention

The present invention has made it possible to produce a three-dimensional structure of cartilage cells that can sufficiently differentiate into a cartilage and that can secrete extracellular matrix. By further culturing this three-dimensional structure in the presence of bone morphogenetic proteins using a bioreactor, cartilage cell tissue can be obtained.

### BRIEF DESCRIPTION OF DRAWINGS

[Figure 1] A view showing schedules for induction of differentiation into cartilage cells.
[Figure 2] Views showing a three-dimensional structure and cartilage tissue obtained when cell aggregates were stacked on the 13th-16th day after the start of induction of differentiation into cartilage cells.
[Figure 3] Views showing three-dimensional structures and cartilage tissues obtained when cell aggregates were stacked on the 3rd or 6th day after the start of induction of differentiation into cartilage cells.
[Figure 4] Views showing three-dimensional structure and cartilage tissue obtained when cell aggregates were stacked on the 9th or 10th day after the start of induction of differentiation into cartilage cells.

### DESCRIPTION OF EMBODIMENTS

Various studies have been conducted on the methods for inducing differentiation of iPS cells into cartilage cells. For example, as a protocol of the Kyoto University, a method of directly producing a mass of cartilage cells by introducing 10 ng/ml TGFB1, 10 ng/ml BMP-2, 10 ng/ml GDF5, and 10 ng/ml bFGF into iPS cells (Non-patent literature 1) is known. According to this method, the mass exhibits features of cartilage tissue including generation of extracellular matrix and thus the method is useful for the treatment of partial defect of cartilage tissue, etc.

However, in order to use the cartilage cell masses for the treatments such as regeneration of a wide curved joint surface, it is necessary to make them into a three-dimensional structure of cartilage cells.

Therefore, in the present invention, the present inventor has found an optimal condition for three-dimensional fabrication using a bio-3D printer where cartilage cells are derived from iPS by going through multiple stages.

The present invention relates to a three-dimensional structure of cartilage cells, which is obtained by stacking cell aggregates obtained by culturing mesenchymal stem cells in a medium that induces differentiation into cartilage cells, on the 9th or 10th day after the start of culture, and to a method for producing the same.

The present invention further relates to cartilage tissue obtained by further culturing the above three-dimensional structure in the presence of bone morphogenetic protein, and to a method for producing the same.

### 1. Mesenchymal stem cells

According to the present invention, cells that are to be cultured using a medium that induces differentiation into cartilage cells are mesenchymal stem cells (MSCs). MSCs are pluripotent cells with self-replication and differentiation ability, which have little risk of tumorigenesis, making them a promising tool for cellular and regenerative medicine.

The source of the MSCs used in the present invention is not limited, and examples thereof include fat, bone marrow, umbilical cord, pluripotent stem cells, and deciduous dental pulp. MSCs are also commercially available and can be obtained from ATCC, Evercyte, CellSource Co., Ltd., Gene Techno Science, etc.

Among the above sources of MSCs, pluripotent stem cells are stem cells that have the pluripotency to differentiate into all cell types existing in the living body and that have proliferation potential, and examples thereof include embryonic stem (ES) cells and induced pluripotent stem (iPS) cells. Preferred pluripotent stem cells are iPS cells.

ES cells are stem cells established from cell masses of early embryos of mammals such as human and mice. ES cells can be established by taking out an inner cell mass from a blastocyst of a fertilized egg of a target mammal and culturing the inner cell mass on a fibroblast feeder layer. Cells can be maintained by passage culture using a culture solution supplemented with substances such as leukemia inhibitory factor (LIF) and basic fibroblast growth factor (bFGF). Human ES cell lines are also available from the Institute for Frontier Life and Medical Sciences, Kyoto University (Kyoto, Japan).

Induced pluripotent stem (iPS) cells are artificial stem cells derived from somatic cells, which have pluripotency and proliferation potential by self-replication almost equivalent to those of ES cells, and can be fabricated by introducing a specific initialization factor into somatic cells (Yamanaka S. et al., Cell, 126: 663-676, 2006; Okita K. et al., Nature 448, 2007; WO2007/069666; etc.). Examples of the gene included in the initialization factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. While these initialization factors can be used alone or in suitable combination, Oct3/4, Sox2, Klf4, and c-Myc are preferable.

Methods for inducing iPS cells from somatic cells are well known (Yamanaka S. et al., Cell, 126: 663-676, 2006; Okita K. et al., Nature 448, 2007; WO2007/069666, etc.).

Examples of the tissue from which the iPS cells used in the present invention are derived include an articular cartilage, bone, adipose tissue, ligament, tendon, tooth, auricle, nose, liver, pancreas, blood vessel, nerve, and heart. Spheroids do not necessarily have to be formed as aggregates of a single cell type, but may include multiple cell types other than iPS cells, for example, undifferentiated cells such as umbilical cord blood-derived cells or differentiated cells thereof, as long as spheroids are formed.

According to the present invention, examples of the method for inducing mesenchymal stem cells (MSCs) from pluripotent stem cells include a method of culturing iPS cells in the presence of a factor such as TGFB1. The period of the induction of differentiation from pluripotent stem cells into MSCs is, for example, 10 days. In this case, the differentiation can take place via neural crest stem cells (NCCs).

Moreover, MSCs derived from iPS cells are also available from Kyoto University.

### 2. Differentiation into cartilage cells and formation of cell aggregates

When a cell suspension of MSCs obtained as described above is seeded onto a plate for fabricating cell aggregates (spheroid plate) and cultured using a differentiation induction medium, MSCs are induced to gradually differentiate into cartilage cells. At the same time, cells aggregate with each other to form cell aggregates (spheroids).

In the present invention, growth factors can be used as the factors to be contained in the differentiation induction medium. Examples of the growth factors include platelet-derived growth factor (PDGF), transforming growth factor-β (TGFβ), and bone morphogenetic protein (BMP) factors, and these factors are contained in the medium that is used during a predetermined period to induce differentiation of MSCs into cartilage cells.

PDGF is a liquid factor stored in platelet alpha-granules. PDGF promotes differentiation and proliferation of pluripotent stem cells into cartilage cells.

There are four known genes encoding PDGF (A-chain, B-chain, C-chain, and D-chain), and there are four homodimers and one heterodimer, AB, as PDGFs with biological activity. AA, AB, and BB undergo proteolytic modification in the cytoplasm and are secreted in mature forms with a molecular weight of about 30,000, and CC and DD are secreted while retaining the CUB domain which inhibits receptor binding.

TGFβ exists in three isoforms (TGFβ1, β2, and β3) in mammals, and structurally similar members of the TGFβ superfamily include activin, BMP (bone morphogenesisinducing factors), etc. Recent studies have shown that TGFβ also contributes to inhibition of proliferation, cell differentiation, induction of apoptosis, etc. for many cell types. For example, it has been reported that TGFβ promotes the proliferation of osteoblasts and the synthesis and proliferation of connective tissues such as collagen, while it has an inhibitory effect on the proliferation of epithelial cells and osteoclasts.

According to the present invention, TGFβ3 is preferably used.

Here, the first day when the cell suspension is seeded onto the spheroid plate, i.e., the first day of culture using a differentiation induction medium, is the start day (Day 0). After Day 0, for example, a medium containing PDGF is used, and TGFβ is added after Day 6.

When MSCs are cultured using a differentiation induction medium containing PDGF, they gradually differentiate into cartilage cells and form spheroids around the 3rd day (Day 3). The formed spheroids are stacked to produce a three-dimensional structure.

According to the present invention, the spheroids are stacked on the 9th or 10th day (Day 9 or Day 10) after the start of culture of the spheroid plate formation using the differentiation induction medium containing PDGF.

According to the present invention, cartilage cells refer to cells that generate extracellular matrix such as collagen that constitutes a cartilage, or to precursor cells thereof. In addition, such cartilage cells may also be cells that express a cartilage cell marker such as type II collagen (COL2A1) or SOX9. According to the present invention, COL2A1 comprises genes having the nucleotide sequences registered under NCBI accession numbers NM _001844 or NM_033150 for humans and NM _001113515 or NM_031163 for mice, proteins encoded by these genes, and naturally occurring mutants having the functions thereof. According to the present invention, SOX9 comprises genes having the nucleotide sequences registered under NCBI accession numbers NM_000346 for humans and NM_011448 for mice, proteins encoded by these genes, and naturally occurring mutants having the functions thereof.

### 3. Spheroid stacking

A method for fabricating a three-dimensional structure of cells by arranging cells in a predetermined three-dimensional space is known (WO2008/123614). According to this method, needle-like bodies are arranged on a substrate like a Kenzan (spiky flower frog), and cell aggregates (spheroids) are arranged by sticking the needle-like bodies into the cell aggregates.

According to the present invention, a three-dimensional structure (3D structure) is fabricated by stacking spheroids using the above method. Since an automatic stacking robot for realizing the above method is already known (Bio-3D Printer "Regenova" (registered trademark), Cyfuse Biomedical K.K.), a three-dimensional structure can also be fabricated using this robot.

The number and shape of the arranged spheroids are not particularly limited, and they can be determined freely.

After spheroids are stacked, they are cultured in the presence of bone morphogenetic protein (BMP) to form cartilage tissue. The reaction vessel that is used to grow the three-dimensional structure into cartilage tissue is called a bioreactor.

BMPs are a group of proteins that have been identified as molecules that induce and promote differentiation of bone tissue and cartilage. BMPs belong to the TGFβ superfamily, bind to type I and II receptor dimers, and transduce signals to the nucleus through phosphorylation of the transcription factor SMAD.

BMPs belonging to the TGFβ superfamily can be classified into BMP2/4 group (BMP2, BMP4), OP-1 group (BMP5, BMP6, BMP7, BMP8a, BMP8b), BMP9 group (BMP9, BMP10), and GDF5 group (GDF5, GDF6, GDF7).

### EXAMPLES

Hereinafter, the present invention will be described more specifically by means of examples. The scope of the present invention, however, should not be limited to these examples.

### [Example 1]

### Method

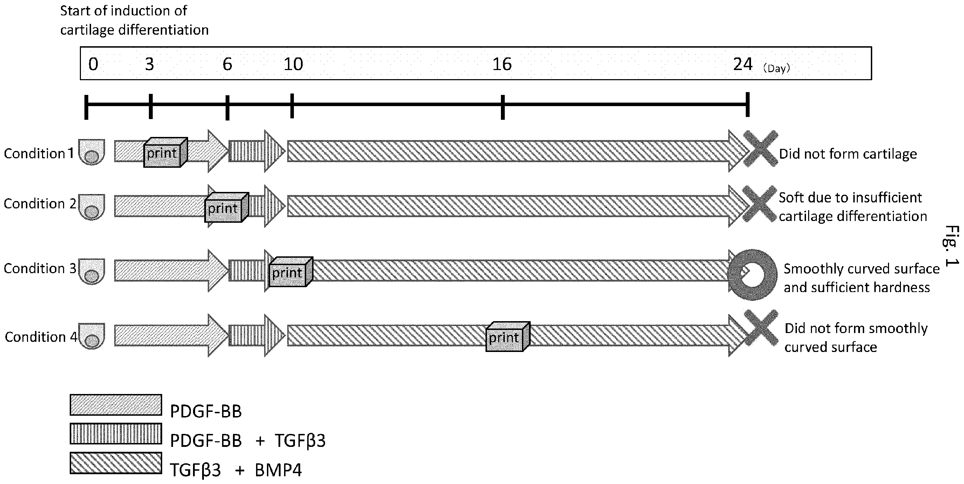

Schedules for induction of differentiation of MSCs into cartilage cells, spheroid formation, and stacking are shown in Figure 1.

An example of fabricating a three-dimensional cartilage cell structure will be described using, as the starting material, mesenchymal stem cells (hereinafter referred to as iMSCs) that have gone through induction of differentiation of human iPS cells provided by Kyoto University into neural crest stem cells.

iMSCs were seeded onto SUMILON (registered trademark) Prime Surface (registered trademark) 96 (Sumitomo Bakelite Co., Ltd., spheroid plate) at 4.5 × 10⁴ cells per well to form spheroids using an induction medium obtained by supplementing chondrogenic basal medium PT-3925 (Lonza) with differentiation induction supplement PT-4121.

The day when iMSCs were seeded onto the spheroid plate was designated as Day 0, and the medium was changed approximately every 3 days. A differentiation induction medium supplemented with PDGF was used from Day 0 to Day 6, a differentiation induction medium + PDGF + TGFβ3 was used from Day 6 to Day 10, and a differentiation induction medium + TGFβ3 + BMP-4 was used from Day 10 to the last day (24).
Platelet-Derived Growth Factor BB: PDGF-BB (R&D Systems, Inc.) 520-BB-050
Transforming Growth Factor-β3: TGF-β3 (PeproTech, Inc.) 100-36E
Bone Morphogenetic Protein 4: BMP-4 (R&D Systems, Inc.) 314-BP-050

The cell masses (spheroids) were placed in the bio-3D printer and stacking (printing) took place under four different conditions for three-dimensional fabrication.
Condition 1: Day 3
Condition 2: Day 6
Condition 3: Day 10
Condition 4: Day 16

After printing, the spheroids were cultured while they were all stuck on the Kenzan, and on the 24th day, the three-dimensional structure of the cells was removed from the Kenzan and evaluated using pathological tissue, etc.

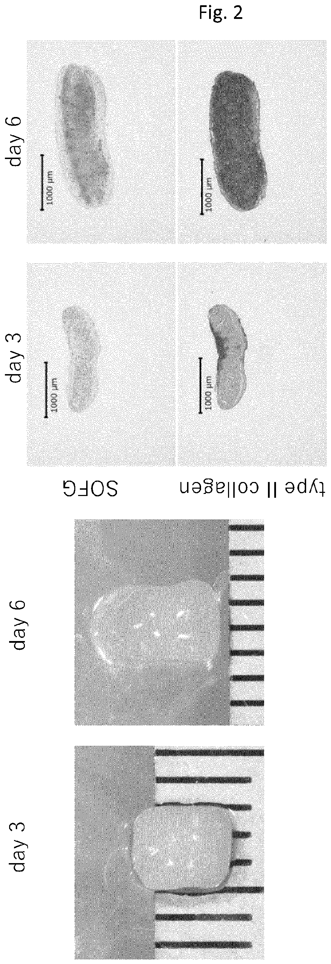

The results are shown in Figures 2 to 4.

Figure 2 shows pictures of the three-dimensional structures obtained under Conditions 1 and 2 on the 24th day.

Both of them fused smoothly, but the structure obtained under Condition 1 had no strength at all and the structure obtained under Condition 2 was as soft or softer than rubber.

The pathological tissues were subjected to cartilage-specific staining, namely, staining that stains proteoglycans red (Safranin-O/Fast-Green staining) and type 2 collagen immunostaining.

Proteoglycans were barely stained under Condition 1. In addition, the expression of type 2 collagen specific to articular cartilage was also low.

Proteoglycans were slightly stained under Condition 2, but very weakly. The expression of type 2 collagen was significant.

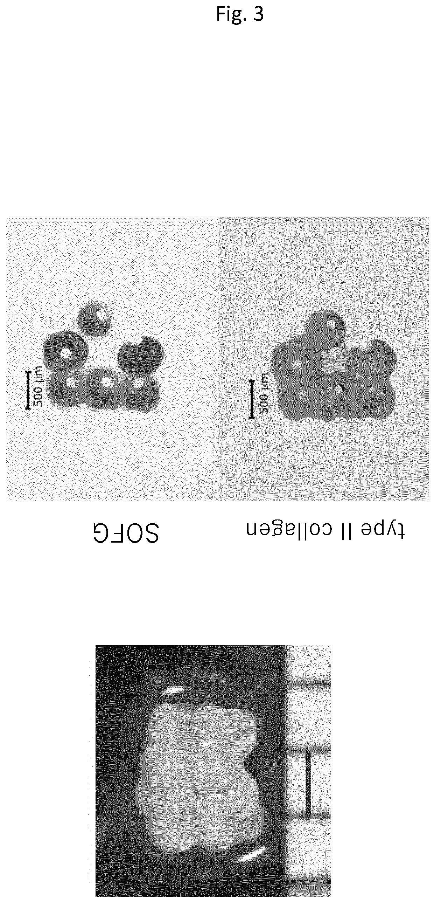

Figure 3 shows pictures of the three-dimensional structure obtained under Condition 4 on the 24th day.

When the culture period of the spheroids was extended, the induction of differentiation into a cartilage progressed too far such that some spheroids could not be penetrated by the needles of the Kenzan.

Under Condition 4, the spheroids did not fuse well with each other, and did not form a smoothly curved surface, resulting in a structure with highly uneven surfaces. This structure could not be expected to serve as a joint even if it was transplanted into an articular cartilage.

Pathologically, the expression of safranin O was prominent and the expression of type 2 collagen was also significant. The structure was very strong.

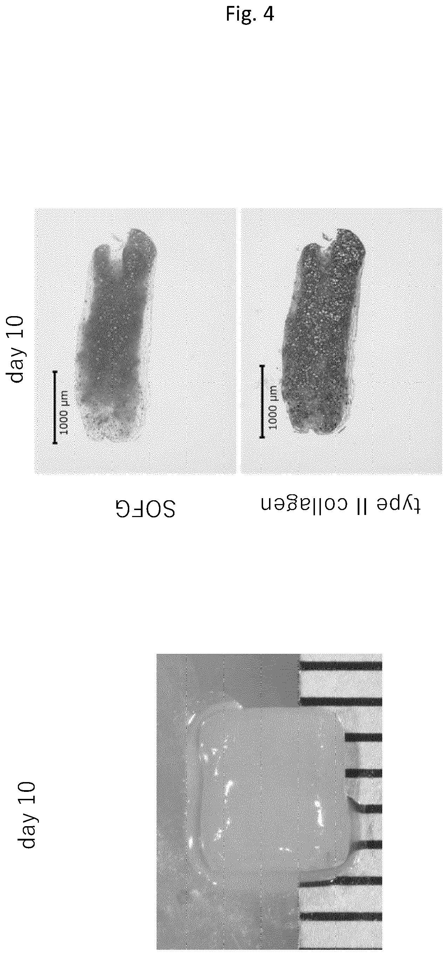

Figure 4 shows pictures of the three-dimensional structure obtained under Condition 3 on the 24th day.

When printing was conducted on the 9th or 10th day after the start of spheroid formation, the spheroids fused well with each other, and a structure with significant strength was obtained. Significant expression of proteoglycans and expression of type 2 collagen were also observed in the pathological tissue sections, confirming that this condition was effective for the regeneration of an articular cartilage (Figure 4).

## Claims

1. A method for producing a three-dimensional structure of cartilage cells, the method comprising stacking cell aggregates obtained by culturing mesenchymal stem cells using a medium that induces differentiation into cartilage cells, on the 9th or 10th day after the start of culture.

2. The method according to claim 1, wherein the mesenchymal stem cells are derived from pluripotent stem cells.

3. The method according to claim 2, wherein the pluripotent stem cells are induced pluripotent stem cells.

4. The method according to any one of claims 1-3, wherein the medium that induces differentiation contains a platelet-derived growth factor.

5. The method according to any one of claims 1-4, wherein the culture is performed in the presence of a transforming growth factor from the 6th day after the start of culture.

6. A method for producing cartilage tissue, the method comprising further culturing a three-dimensional structure produced by the method according to any one of claims 1-5 in the presence of bone morphogenetic protein.

7. A three-dimensional structure of cartilage cells, which is obtained by stacking cell aggregates obtained by culturing mesenchymal stem cells using a medium that induces differentiation into cartilage cells, on the 9th or 10th day after the start of culture.

8. Cartilage tissue obtained by further culturing the three-dimensional structure according to claim 7 in the presence of bone morphogenetic protein.
